# EUROPÄISCHE PATENTSCHRIFT

(11) **EP 1 000 921 B1**
(45) Veröffentlichungstag und Bekanntmachung des Hinweises auf die Patenterteilung: **03.04.2002**
(21) Anmeldenummer: 99118229.6
(22) Anmeldetag: 14.09.1999
(51) Int. Cl.: C07C 45/58, C07C 47/19

(54) **Verfahren zur Herstellung von beta-Hydroxyaldehyden**
Process for the preparation of beta-hydroxyaldehydes
Procédé pour la préparation de beta-hydroxyaldéhydes

(30) Priorität: 12.11.1998 DE 19852104
(43) Veröffentlichungstag der Anmeldung: 17.05.2000
(73) Patentinhaber: Degussa AG, 40474 Düsseldorf (DE)
(72) Erfinder: Weber, Robert, Dr., 63755 Alzenau (DE); Keim, Wilhelm, Prof., 52074 Aachen (DE); Jaeger, Bernd, Dr., 64297 Darmstadt (DE); Haas, Thomas, Dr., 60316 Frankfurt am Main (DE); Vanheertum, Rudolf, Dr., 63796 Kahl (DE)

(56) Entgegenhaltungen:
- EP-A- 0 073 961
- EP-A- 0 455 261
- WO-A-96/10552
- US-A- 5 304 691
- ABU-GNIM C ET AL: "Phosphine oxides as ligands in the hydroformylation reaction" J. ORGANOMET. CHEM. (JORCAI,0022328X);1996; VOL.516 (1-2); PP.235-243, XP002125542 The Institutes for Applied Research and The Department of Chemistry, Ben-Gurion University;Beer Sheva; 84105; Israel (IL)

## Beschreibung

Die Erfindung betrifft ein Verfahren zur Herstellung von β-Hydroxyaldehyden.

β-Hydroxyaldehyde sind als Ausgangsverbindungen für die Herstellung einer Reihe von Produkten, wie zum Beispiel Dicarbonsäuren und deren Derivate, von Bedeutung. Weiterhin können durch die Hydrierung der β-Hydroxyaldehyde die Diole, wie zum Beispiel 1,3-Propandiole, hergestellt werden. 1,3-Propandiole können als Monomerbaustein bei der Herstellung von Polyestern und Polyurethanen, sowie als Ausgangsstoffe für die Synthese cyclischer Verbindungen sowie einer Reihe anderer Stoffe eingesetzt werden.

Es ist bekannt, β-Hydroxyaldehyde herzustellen, indem man 1,2-Oxirane mit Kohlenmonoxid und Wasserstoff in Gegenwart von Katalysatoren umsetzt.

US-A 3,463,819 beschreibt ein Verfahren zur Herstellung von β-Hydroxypropionaldehyd und 1,3-Propandiol, bei dem unter anderem Ethylenoxid in Gegenwart von Toluol als auch in Gegenwart von Diethylether/Benzol, 1,2-bis(diphenylphosphino)ethandicobalthexacarbonyl, Kohlenmonoxid und Wasserstoff miteinander umgesetzt werden.

US-A 3,456,017 beschreibt ein Verfahren zur Herstellung von Propandiol und β-Hydroxypropionaldehyd, bei dem Ethylenoxid, ein tertiär phosphinomodifizierter Kobaltcarbonylkatalysator, Kohlenmonoxid und Wasserstoff miteinander umgesetzt werden.

US-A 5,256,827 beschreibt die Hydroformylierung von Ethylenoxid in Gegenwart von partiell oxidierten tertiär phosphinkomplexierten Kobalt-Carbonyl-Katalysatoren.In diesem Verfahren, das im Hinblick auf die vorliegende Erfindung als nächstliegender Stand der Technik angesehen werden kann, wird ein Bisphosphin zunächst bis zu einem O/P-Verhältnis von nicht größer als 0,5 partiell oxidiert und das so erhaltene Produkt als Ligand eingesetzt.

US-A 5,304,691 lehrt ein ähnliches Verfahren wie das zuvor beschriebene, jedoch wird zusätzlich zu einem Kobaltkatalysator ein Rutheniumkatalysator eingesetzt. Ligand des Co-Carbonyl-Katalysators ist ein Phosphin oder ein partiell oxidiertes Bisphosphin.

Die EP-A 0 073 961 lehrt ein Verfahren zur Hydroformylierung von Olefinen unter Verwendung eines Rhodiumkomplexes mit einem Bisphosphinmonooxid als Ligand. Außer dem Rh-Phosphorligand-Komplex muss zusätzlich freier Phosphorligand anwesend sein - ausweislich Anspruch 2 werden pro Mol Rh 3 bis 80 Mol, ausweislich der Beispiele 1 bis 19 20 bis 40 Mol Phosphorligand eingesetzt.

Die bekannten Verfahren weisen den Nachteil auf, daß eine sehr hohe Menge an Katalysator, bezogen auf das Ethylenoxid, eingesetzt werden muß. Weiterhin müssen Promotoren zugesetzt werden.

Es ist Aufgabe der Erfindung, ein Verfahren zur Herstellung von β-Hydroxyaldehyden, welches diese Nachteile nicht aufweist, zu entwickeln. Insbesondere sollte eine höhere Ausbeute erzielt werden.

Gegenstand der Erfindung ist ein Verfahren zur Herstellung von β-Hydroxyaldehyden, umfassend Umsetzung eines 1,2-Oxirans mit Kohlenmonoxid und Wasserstoff in Gegenwart von Kobaltcarbonylverbindungen, die mit Phosphor-Sauerstoff- oder Stickstoff-Sauerstoff-Liganden modifiziert sind und als Katalysator wirken, das
dadurch gekennzeichnet ist,
dass die Liganden ausgewählt sind aus der Reihe der
Bisphospinmonoxide der allgemeinen Formel (I) 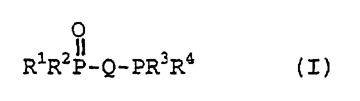 in der
- R¹; R²; R³; R⁴: jeweils verschieden sein können und für einen Alkyl-, Cycloalkyl- oder Arylrest mit bis zu 30 Kohlenstoffatomen oder für -CH₂-OH und
- Q: für eine Alkylidenverbrückung mit 1 bis 20 Kohlenstoffatomen oder eine ein Sauerstoff- oder Stickstoffatom enthaltende Alkyliden-Verbindungen stehen,
Phosphinocarbonsäureverbindungen der allgemeinen Formel (II) und (III) 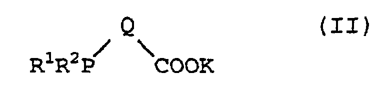 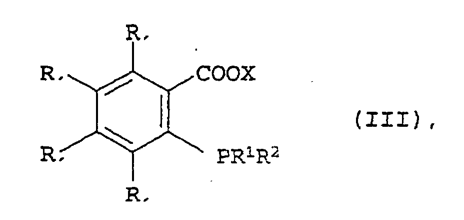 in denen
- R1, R2: jeweils verschieden sein können und für einen Alkyl-, Cycloalkyl- oder Arylrest mit bis zu 30 Kohlenstoffatomen oder für -CH₂-OH,
- R': für Wasserstoff oder einen Alkyl- oder Cycloalkylrest mit bis zu 30 Kohlenstoffatomen Kohlenstoffatomen
- Q: für eine Alkylidenverbrückung mit 1-20, Kohlenstoffatomen und
- X: für Wasserstoff oder eine Alkyl-, Cycloalkyl- oder Arylgruppe stehen,
Aminocarbonsäureverbindungen der allgemeinen Formel (IV) und Pyridincarbonsäureverbindungen der allgemeinen Formel (V) 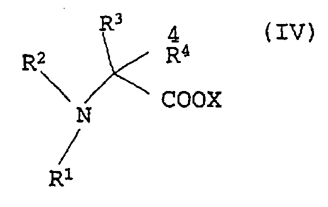 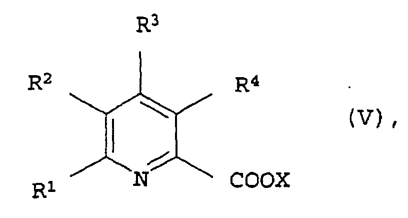 in denen
- R1, R2, R3, R4: jeweils für Wasserstoff,Alkyl-Cycloalkyl- oder Arylreste mit bis zu 30 Kohlenstoffatomen stehen, die zusätzlichen weiteren Funktionalitäten, wie Hydroxyl-, Amino- oder Halogengruppen tragen können,
- R1 und R2: beziehungsweise R3 und R4 zudem jeweils zu einemgemeinsamen Cycloalkylrest mit bis zu 20, insbesondere 4 - 7 Kohlenstoffatomen gehören können und
- X: für Wasserstoff oder für eine Alkyl-, Cycloalkyl- oder Arylgruppe steht.

Erfindungsgemäß werden 1,2-Oxirane (Epoxide) mit Kohlenmonoxid und Wasserstoff in Gegenwart eines mit Phosphor-Sauerstoff- oder Stickstoff-Sauerstoff-Chelatliganden modifizierten Kobalt-Carbonyl-Katalysators hydroformyliert. Die Reaktionsprodukte sind im wesentlichen β-Hydroxyaldehyde (und deren Oligomere) und in kleinen Anteilen die entsprechenden 1,3-Diole. Wenn im weiteren die Bezeichnung "β-Hydroxyaldehyde" verwendet wird, so sind damit sowohl die Monomere als auch die Dimere, wie zum Beispiel 2-(2-Hydroxyethyl)-4-hydroxy-1,3-dioxan im Falle der Hydroformylierung von Etylenoxid, als auch Trimere und höhere Oligomere der entsprechenden β-Hydroxyaldehyde gemeint.

Als 1,2-Oxirane können zum Beispiel solche, die 2 bis 30 Kohlenstoffatome enthalten, eingesetzt werden.

Als 1,2-Oxirane werden bei dem erfindungsgemäßen Verfahren gesättigte Kohlenwasserstoffe mit vicinaler Epoxidgruppe mit bis zu 10 Kohlenstoffatomen eingesetzt werden. Die auch Epoxyalkane genannten Verbindungen können cyclische oder acyclische, langkettige oder verzweigtkettige Strukturen aufweisen.

Acyclische Epoxide können solche Epoxide sein, bei denen die Kohlenstoffatome der Epoxy-Einheit nicht Bestandteil eines carbocyclischen Ringes sind. Derartige Verbindungen. sind zum Beispiel Ethylenoxid, Propylenoxid, Isobutylenoxid, 1,2-Epoxypentan, 1,2-Epoxy-4-methylpentan, 1,2-Epoxyoctan, 3-Cyclohexyl-1,2-epoxypropan, 3,4-Epoxynonan, 1,2-Epoxy-2,2,4-trimethylhexan und 1,2-Epoxydecan.

Cyclische Epoxide können solche Epoxide sein, bei denen die Kohlenstoffatome der Epoxy-Einheit Teil eines carbocyclischen Ringes ist. Derartige Epoxycycloalkane können zum Beispiel sein: Cyclohexenoxid, Cyclopentenoxid, Cyclooctenoxid, 1,2-Epoxy-4-methylcyclohexan, 4,5-Dimethyl-1,2-epoxycyclohexan, 2,3-Epoxy-decahydronaphthalin und 1,2-Epoxy-4-propylcyclohexan.

Im allgemeinen wird die Verwendung von acyclischen Epoxiden bevorzugt. Insbesondere können acyclische Epoxide mit einer Kohlenstoffatomzahl bis zu 6 Kohlenstoffatomen, bei denen die Epoxy-Gruppe endständig, wie zum Beispiel bei einem acyclischen 1,2-Epoxyalkan, ist, eingesetzt werden. Von diesen Verbindungen sind sowohl Ethylenoxid als auch Propylenoxid besonders bevorzugt.

Als Katalysatoren werden gemäß einer Ausführungsform des Verfahrens mit Phosphor-Sauerstoff-Chelatliganden modifizierte Kobalt-Carbonyl-Katalysatoren eingesetzt.

Bei den Chelatliganden der allgemeinen Formel (I) handelt es sich um Phosphin-Phosphinoxide , also Bisphosphinmonoxide

Besonders bevorzugte Stoffe entsprechend der Formel (I) sind Bis(diphenylphosphino)methanmonoxid(dppmO) und 1,2-Bis(diphenylphosphino)ethanmonoxid(dppeO).

Diese Verbindungen sind bekannt aus US-A 4,429,161 und US-A 3,426,021, sowie N.A. Bondarenko, Synthesis 1991, 125.

Die erfindungsgemäß eingesetzten Bisphosphinmonoxide der Formel (I) sind definierte Verbindungen

Als Liganden können weiterhin zum Beispiel aliphatische und aromatische Phosphinocarbonsäuren und deren Ester gemäß in den allgemeinen Formeln (II) und (III) in Frage kommen.

Diese Verbindungen sind bekannt aus K. Issleib und G. Thomas in Chem. Ber. 1960, (93), 803 bis 808. Sie können hergestellt werden nach O. Stelzer et al., J. Organomet. Chem. 1996, 522, 69-76 sowie nach Rauchfuss, Inorg. Synth., 21, 1982.

Besonders bevorzugte Stoffe entsprechend der Formel (II) sind Diphenylphosphinoessigsäure und Diphenylphosphinoessigsäuremethylester.

Besonders bevorzugte Stoffe entsprechend der Formel (III) sind o-Diphenylphosphinobenzoesäure und o-Diphenylphosphinobenzoesäuremethylester.

Des weiteren werden bei dem erfindungsgemäßen Verfahren mit Stickstoff-Sauerstoff-Chelatliganden modifizierte Kobalt-Carbonyl-Verbindungen eingesetzt.

Als Liganden können di-N-substituierte Aminocarbonsäuren sowie deren Ester gemäß der allgemeinen Formel (IV) eingesetzt werden.

Besonders bevorzugte Stoffe, die der Formel (IV) entsprechen sind N,N-Dimethylglycin beziehungsweise dessen Ethylester.

Die Verbindungen, welche der Formel (IV) entsprechen, können im Fall R³ ≠ R⁴ in Form der reinen Enantiomere hergestellt werden, um auf diese Weise eine chirale Information auf entsprechende chirale Epoxide zu übertragen.

Weiterhin geeignete Liganden sind Stoffe, gemäß der allgemeinen Formel (V).

Besonders bevorzugte Stoffe, die der Formel (V) entsprechen, sind Picolinsäuren (Pyridin-2-carbonsäure) beziehungsweise deren Ethylester.

In bevorzugten Liganden der Formeln (I) bis (IV) stehen R¹, R², R³, R⁴ für Wasserstoff oder einen Alkyl-, Cycloalkyl- oder Arlyrest mit bis zu 12 Kohlenstoffatomen. Die Alkylidenverbrückung Q in den Formeln (I) und (II) enthält vorzugsweise 1 bis 10 Kohlenstoffatome. In Liganden der Formel (III) ist R'bevorzugt Wasserstoff.

Als Kobaltverbindungen können beispielsweise Salze mit organischen oder anorganischen Anionen verwendet werden. Bevorzugt sind Kohlenmonoxid-Komplexe des Kobalts. Eine besonders bevorzugte Kobaltverbindung ist Dikobaltoctacarbonyl.

Für die Herstellung der mit Phosphor-Sauerstoff- oder P-O und N-O-Chelatliganden modifizierten Kobalt-Carbonyl-Katalysatoren gibt es eine Reihe von Verfahren. Zum Beispiel ist es möglich, Kobalt-Komplexe, wie zum Beispiel Dikobaltoctacarbonyl und einen geeigneten Liganden, zusammen zu erwärmen. Der Ligand wird die entsprechende Anzahl an Carbonylfunktionen ersetzen und so den gewünschten Katalysatorvorläufer ergeben. Es ist zu berücksichtigen, daß eine Koordination der Liganden sowohl über das Phosphor- beziehungsweise Stickstoffatom als auch über das Sauerstoffatom möglich ist. Führt man diese Reaktion in einem geeigneten Lösungsmittel durch, so ist es möglich, den Komplex durch Kühlen in kristalliner Form zu erhalten.

Eine weitere Möglichkeit ist die in situ Herstellung. Dazu werden Kobalt-Verbindungen, wie zum Beispiel Dikobaltoctacarbonyl, und ein geeigneter Ligand, nacheinander in die Reaktionslösung gegeben. Unter den gewählten Reaktionsbedingungen bildet sich der katalytisch aktive Komplex.

Das Verhältnis Ligand zu Kobalt liegt bevorzugt zwischen 0,1 : 1 und 5 :1.

Bezogen auf 1 mol Epoxid kann man zum Beispiel 0,5 bis 0,0001 mol Katalysator verwenden. Vorzugsweise liegt diese Menge bei 0,2 bis 0,002 mol.

Das erfindungsgemäße Hydroformylierungsverfahren kann bei Temperaturen im Bereich von 30 - 150 °C und Drücken zwischen 50 und 300 bar durchgeführt werden. Vorzugsweise arbeitet man bei 70 - 120 °C und Drücken zwischen 90 und 200 bar.

Das erfindungsgemäße Verfahren beinhaltet die Reaktion eines Epoxids in Gegenwart eines Katalysators mit Wasserstoff und Kohlenmonoxid. Dabei wird ein Verhältnis von Wasserstoff zu Kohlenmonoxid zwischen 6 : 1 und 1 : 1 bevorzugt.

Als Lösungsmittel können solche Verbindungen eingesetzt werden, die bei den gegebenen Temperaturen und Drücken flüssig und inert gegen die Reaktanden und Katalysatoren sind. Dies sind beispielsweise aliphatische und aromatische Kohlenwasserstoffe, chlorhaltige aliphatische und aromatische Kohlenwasserstoffe sowie lineare und cyclische Ether. Bevorzugte Lösungsmittel sind Benzol, Toluol, Diethylether, MTBE und Tetrahydrofuran.

Das erfindungsgemäße Verfahren weist die folgenden Vorteile auf:
Die Hydroformylierung von 1,2-Epoxiden kann so durchgeführt werden, daß die β-Hydroxyaldehyde in hohem Grade selektiv und in hohen Ausbeuten gebildet werden.
Die Verwendung von Promotoren ist nicht notwendig.
Das erfindungsgemäße Verfahren ermöglicht eine Verbesserung der Ausbeute (60 - 70 %) und der Selektivität (80 - 90 %).
Die Ausbeuten bei der Hydroformylierung höherer Epoxide liegen deutlich über denen, die mit bekannten Verfahren erreicht werden können.
Gegenüber dem bekannten Verfahren gemäß US-A 3,463,819 hat das erfindungsgemäße Verfahren den Vorteil, daß eine wesentlich geringere Katalysatormenge benötigt wird. So wird anstelle von 5 g Katalysator für 3 g Ethylenoxid nur 400 mg Katalysator für 2,2 g Ethylenoxid benötigt.
Bei dem Verfahren gemäß US-A 5,256,827 wird kein definiertes Phosphinoxid eingesetzt.
Das erfindungsgemäße Verfahren, bei welchem eine definierte Phosphinoxid-Verbindung eingesetzt wird, erzielt deutlich höhere Ausbeuten, obwohl keine Promotoren eingesetzt werden.

### Beispiele

### Beispiel 1

In einen mit Inertgas gespülten 100 ml Stahlautoklaven, der mit einem temperierbaren Tropftrichter mit Druckausgleich ausgestattet ist, werden 68,4 mg (0,2 mmol) Dikobaltoctacarbonyl, 321,6 mg (0,8 mmol) (gelöscht: 1,2-) Bis(diphenylphosphino)methanmonoxid(dppmO) und 20 ml Toluol vorgelegt. In den auf 0 °C gekühlten Tropftrichter werden 2,20 g (50 mmol) flüssiges Ethylenoxid und 1,00 g Diethylenglykoldimethylether (Standard für die GC-Analytik) gegeben. Der Autoklav wird nun verschlossen und es werden 100 bar Synthesegas (Kohlenmonoxid-Wasserstoff-Gemisch im Verhältnis 1 : 1) aufgepreßt. Die Reaktionslösung wird nun insgesamt 4 h auf 100 °C erwärmt. Nach 1 h wird die Ethylenoxidlösung zu der Katalysatorlösung gegeben. Nach Ablauf der Reaktionszeit von 3 h wird der Autoklav auf 5 °C gekühlt, dann entspannt und geöffnet. Die Reaktionslösung wird entnommen, und der Autoklav wird noch zweimal mit je 3 ml Methanol nachgespült. Die vereinigten organischen Phasen werden gaschromatographisch untersucht. Die Ergebnisse sind in der Tabelle 1 aufgeführt.

### Beispiele 2 - 6

Es wird, wie in Beispiel 1 beschrieben, verfahren. Anstelle von Bis(diphenylphosphino)methanmonoxid wird o-Diphenylphosphinobenzoesäuremethylester als Katalysator verwendet. Zusätzlich wird das Verhältnis von Ligand zu Kobalt und der Reaktionsdruck variiert. Die Ergebnisse sind in der Tabelle 1 aufgeführt.

**Tabelle 1**

| Beispiel | p [bar] | Verhältnis Ligand / Co | Ausbeute 3-HPA [%] | Selektivität [%] |
|---|---|---|---|---|
| 1 | 100 | 2,0 | 65 | 90 |
| 2 | 150 | 0,25 | 28 | 66 |
| 3 | 150 | 0,5 | 24 | 60 |
| 4 | 150 | 1,0 | 35 | 61 |
| 5 | 150 | 1,5 | 48 | 79 |
| 6 | 150 | 2,0 | 56 | 83 |

### Beispiel 7

Es wird wie in Beispiel 5 beschrieben verfahren. Als Edukt werden jedoch 2,9 g (50 mmol) Propylenoxid verwendet. Die Ausbeute an 3-Hydroxybutyraldehyd beträgt 45 %.

### Beispiel 8-10

Es wird wie in Beispiel 4 beschrieben verfahren. Als Edukt wird jedoch 2,9 g (50 mmol) Propylenoxid verwendet. An Stelle des o-Diphenylphosphinobenzoesäuremethylesters werden Picolinsäure (Beispiel 8), Picolinsäuremethylester (Beispiel 9) beziehungsweise N,N-Dimethylglycinethylester (Beispiel 10) verwendet. Die Ergebnisse sind in Tabelle 2 aufgeführt.

**Tabelle 2**

| Beispiel | Selektivität zu 3-Hydroxyaldehyden / % | Ausbeute an 3-Hydroxybutyraldehyd / % |
|---|---|---|
| 8 | 65 | 36 |
| 9 | 57 | 34 |
| 10 | 50 | 34 |

### Beispiel 11

Es wird wie in Beispiel 10 verfahren, allerdings wird die Reaktionszeit auf 1 h begrenzt. Die Ausbeute an 3-Hydroxybuthyraldehyd beträgt 51 %.

## Patentansprüche

1. Verfahren zur Herstellung von β-Hydroxyaldehyden, umfassend Umsetzung eines 1,2-Oxirans mit Kohlenmonoxid und Wasserstoff in Gegenwart von Kobaltcarbonylverbindungen, die mit Phosphor-Sauerstoff- oder Stickstoff-Sauerstoff-Liganden modifiziert sind uns als Katalysator wirken,
**dadurch gekennzeichnet,**
**dass** die Liganden ausgewählt sind aus der Reihe der Bisphospinmonoxide der allgemeinen Formel (I) 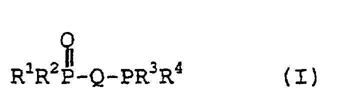 in der
R¹; R²; R³; R⁴ jeweils verschieden sein können und für einen Alkyl-, Cycloalkyl- oder Arylrest mit bis zu 30 Kohlenstoffatomen oder für -CH₂-OH und
Q für eine Alkylidenverbrückung mit 1 bis 20 Kohlenstoffatomen oder eine ein Sauerstoff- oder Stickstoffatom enthaltende Alkyliden-Verbindungen stehen
Phosphinocarbonsäureverbindungen der allgemeinen Formel (II) und (III) 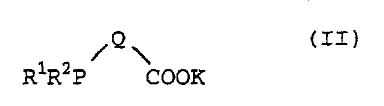 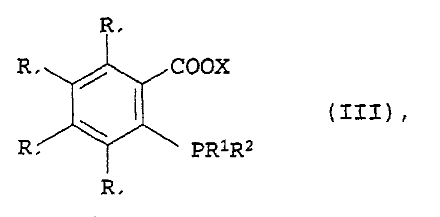 in denen
R1, R2 jeweils verschieden sein können und für einen Alkyl-, Cycloalkyl- oder Arylrest mit bis zu 30 Kohlenstoffatomen oder für -CH₂-OH,
R' für Wasserstoff oder einen Alkyl- oder Cycloalkylrest mit bis zu 30 Kohlenstoffatomen Kohlenstoffatomen
Q für eine Alkylidenverbrückung mit 1-20, Kohlenstoffatomen und
X für Wasserstoff oder eine Alkyl-, Cycloalkyl- oder Arylgruppe stehen,
Aminocarbonsäureverbindungen der allgemeinen Formel (IV) und Pyridincarbonsäureverbindungen der allgemeinen Formel (V) 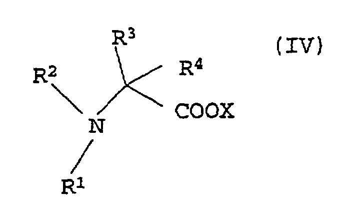 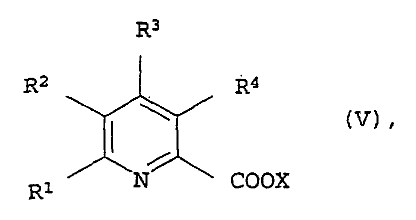 in denen
R1, R2, R3, R4 jeweils für Wasserstoff oder einen Alkyl-, Cycloalkyl- oder Arylresten mit bis zu 30 Kohlenstoffatomen stehen, die zusätzlichen weiteren Funktionalitäten, wie Hydroxyl-, Amino- oder Halogengruppen tragen können.
R1 und R2 beziehungsweise R3 und R4 zudem jeweils einem gemeinsamen Cycloalkylrest mit bis zu 20, insbesondere 4 - 7 Kohlenstoffatomen gehören können und
X für Wasserstoff oder für eine Alkyl-, Cycloalkyl- oder Arylgruppe steht.

2. Verfahren nach Anspruch 1,
**dadurch gekennzeichnet,**
**dass** man einen Liganden der Formel (I), (II), (III), (IV) oder (V) verwendet, worin nach Maßgabe der im Auspruch 1 gegebenen Formeldefinitionen R¹, R², R³ und R⁴ jeweils für Wasserstoff, einen Alkyl-, Cycloalkyl- oder Arylrest mit bis zu 12 Kohlenstoffatomen stehen.

3. Verfahren nach Anspruch 1 oder 2,
dadurch gekennzeichent,
dass man einen Liganden der Formel (I) oder (II), worin Q für eine Alkylidenverbrückung mit 1 bis 10 Kohlenstoffatomen steht, insbesondere Bis(diphenylphosphino)methanmonoxid, 1,2-Bis(diphenylphosphino)ethanmonoxid, Diphenylphosphinoessigsäure oder deren Methylester verwendet.

4. Verfahren nach Anspruch 1 oder 2,
**dadurch gekennzeichnet, dass** man einen Liganden der Formel (III), worin R' für Wasserstoff steht, verwendet.

5. Verfahren nach Anspruch 1 oder 2,
**dadurch gekennzeichnet,**
**dass** man als Liganden Pyridin-2-carbonsäure oder N,N-Dimethylglycin oder einen Alkylester dieser Carbonsäuren verwendet.

6. Verfahren nach einem der Ansprüche 1 bis 5,
dadurch gekennzeichent, dass das Verhältnis Ligand zu Kobalt im Bereich von 1 bis 5 liegt und insbesondere 1,5 bis 2 beträgt.

## Claims

1. Process for the production of β-hydroxyaldehydes, comprising reaction of a 1,2-oxiran with carbon monoxide and hydrogen in the presence of cobalt carbonyl compounds, which are modified with phosphorus-oxygen or nitrogen-oxygen ligands and act as a catalyst,
**characterised in that**
the ligands are selected from the series of
bisphosphine monoxides having the general formula (I) 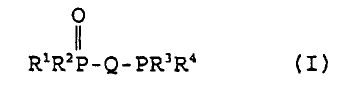 in which
R¹; R²; R³; R⁴ can each be different and stand for an alkyl, cycloalkyl or aryl radical with up to 30 carbon atoms or for -CH₂-OH and
Q stands for an alkylidene bridge with 1 to 20 carbon atoms or an alkylidene compound containing an oxygen or nitrogen atom
phosphinocarboxylic acid compounds having the general formula (II) and (III) 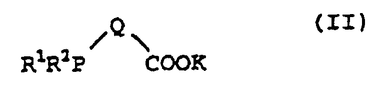 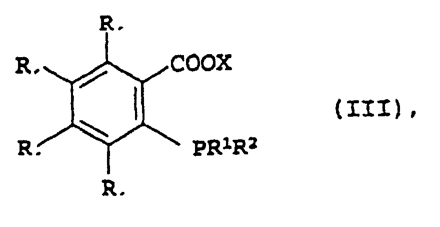 in which
R1, R2 can each be different and stand for an alkyl, cycloalkyl or aryl radial with up to 30 carbon atoms or for -CH₂-OH,
R' stands for hydrogen or an alkyl or cycloalkyl radical with up to 30 carbon atoms
Q stands for an alkylidene bridge with 1-20 carbon atoms and
X stands for hydrogen or an alkyl, cycloalkyl or aryl group,
aminocarboxylic acid compounds having the general formula (IV) and pyridine carboxylic acid compounds having the general formula (V) 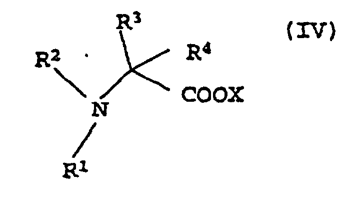 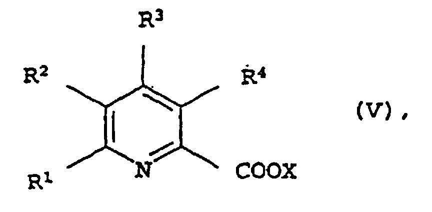 in which
R1, R2, R3, R4 each stand for hydrogen or an alkyl, cycloalkyl or aryl radical with up to 30 carbon atoms, which can carry other additional functionalities such as hydroxyl, amino or halogen groups.
R1 and R2 or R3 and R4 can moreover each belong to a common cycloalkyl radical with up to 20, particularly 4 to 7 carbon atoms and
X stands for hydrogen or for an alkyl, cycloalkyl or aryl group.

2. Process according to claim 1,
**characterised in that**
a ligand having formula (I), (II), (III), (IV) or (V) is used, wherein according to the formula definitions specified in claim 1, R¹, R², R³ and R⁴ each stand for hydrogen, an alkyl, cycloalkyl or aryl radical with up to 12 carbon atoms.

3. Process according to claim 1 or 2,
**characterised in that**
a ligand having formula (I) or (II) is used, wherein Q stands for an alkylidene bridge with 1 to 10 carbon atoms, particularly bis(diphenylphosphino)methane monoxide, 1,2-bis(diphenylphosphino)ethane monoxide, diphenyl phosphinoacetic acid or methyl esters thereof.

4. Process according to claim 1 or 2,
**characterised in that** a ligand having formula (III) is used, wherein R' stands for hydrogen.

5. Process according to claim 1 or 2,
**characterised in that**
pyridine-2-carboxylic acid or N,N-dimethylglycine or an alkyl ester of these carboxylic acids is used as ligand.

6. Process according to one of claims 1 to 5,
**characterised in that** the ratio of ligand to cobalt is in the range from 1 to 5 and is in particular 1.5 to 2.

## Revendications

1. Procédé de préparation de β-hydroxyaldéhydes qui comprend la réaction d'un 1,2-oxirane ave le monoxyde de carbone et l'hydrogène, en présence de composés du cobaltcarbonyle modifiés par des ligands phosphore-oxygène ou azote-oxygène et agissant comme catalyseur,
**caractérisé en ce que**
les ligands sont choisis dans la série
- des monoxydes de bisphosphine de formule générale (I): 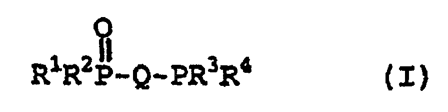 dans laquelle
R¹, R², R³ et R⁴ peuvent être respectivement différents et représentent un reste alkyle, cycloalkyle ou aryle ayant jusqu'à 30 atomes de carbone ou -CH₂-OH et
Q est un élément de pontage alkylidène ayant de 1 à 20 atomes de carbone ou des composés alkylidène contenant un atome d'oxygène ou un atome d'azote,
- des composés de l'acide phosphinocarboxylique de formules générales (II) et (III) : 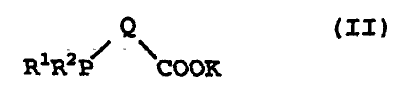 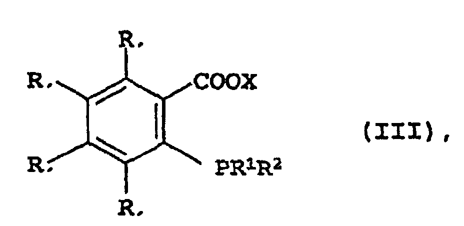 dans lesquelles
R¹ et R² respectivement peuvent être différents et représentent un reste alkyle, cycloalkyle ou aryle ayant jusqu'à 30 atomes de carbone ou représentent -CH₂-OH,
R' représente de l'hydrogène ou un reste alkyle, ou cycloalkyle ayant jusqu'à 30 atomes de carbone,
Q représente un élément de pontage alkylidène, ayant de 1 à 20 atomes de carbone, et
X représente de l'hydrogène ou un groupe alkyle cycloalkyle ou aryle,
- des composés acides aminocarboxylique de formule générale IV et des composés acides pyridine carboxyliques de formule générale V : 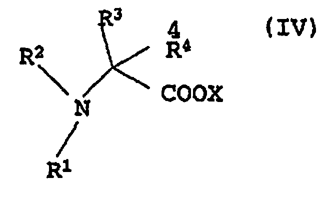 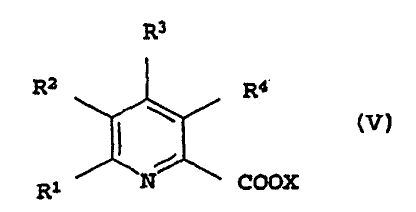 dans laquelle
R¹, R², R³ et R⁴ respectivement représentent de l'hydrogène ou un reste alkyle, cycloalkyle ou aryle ayant jusqu'à 30 atomes de carbone, qui peuvent porter en supplément d'autres fonctionnalités comme des groupes hydroxyle, amino ou halogène,
R¹ et R² ou R³ et R⁴ en outre peuvent à chaque fois appartenir à un reste cycloalkyle commun ayant jusqu'à 20 - en particulier de 4 à 7 - atomes de carbone, et
X représente de l'hydrogène ou un groupe alkyle, cycloalkyle ou aryle.

2. Procédé selon la revendication 1,
**caractérisé en ce qu'**
on utilise un ligand de formules (I), (II), (III), (IV) ou (V) dans lesquelles selon la précision des définitions de formule donnée à la revendication 1 R¹, R², R³ et R⁴ respectivement représentent un hydrogène, un reste alkyle, cycloalkyle ou aryle ayant jusqu'à 12 atomes de carbone.

3. Procédé selon la revendication 1 ou la revendication 2,
**caractérisé en ce qu'**
on utilise un ligand de formule (I) ou de formule (II) dans lesquelles Q représente un élément de pontage, en particulier le mono-oxyde de bis diphénylphosphino)méthane, le monoxyde de 1,2-bis(diphénylphosphino)éthane, l'acide diphénylphosphinoacétique ou son ester méthylique.

4. Procédé selon la revendication 1 ou la revendication 2,
**caractérisé en ce qu'**
on utilise un ligand de formule (III) dans laquelle R' représente de l'hydrogène.

5. Procédé selon la revendication 1 ou la revendication 2,
**caractérisé en ce qu'**
on utilise comme ligand de l'acide pyridine-2-carboxylique ou la N,N-diméthylglycine ou un ester d'alkyle de ces acides carboxyliques.

6. Procédé selon l'une des revendications 1 à 5,
**caractérisé en ce que**
le rapport ligand au cobalt se situe dans la zone de 1 à 5 et en particulier s'élève à 1,5 à 2.
